# FASCICULE DE BREVET EUROPEEN

(11) **EP 0 607 295 B1**
(45) Date de publication et mention de la délivrance du brevet: **09.04.1997**
(21) Numéro de dépôt: 92921712.3
(22) Date de dépôt: 05.10.1992
(51) Int. Cl.: G02B 21/00

(54) **APPAREIL POUR OBSERVER IN VIVO LA STRUCTURE MICROSCOPIQUE DE LA PEAU OU D'UN TISSU SIMILAIRE**
VORRICHTUNG FÜR DIE IN VIVO BEOBACHTUNG DER MIKROSKOPISCHEN STRUKTUREN DER HAUT ODER EINES ÄHNLICHEN GEWEBES
APPARATUS FOR IN VIVO OBSERVATION OF THE MICROSCOPIC STRUCTURE OF THE SKIN OR SIMILAR TISSUE

(30) Priorité: 11.10.1991 FR 9112541
(43) Date de publication de la demande: 27.07.1994
(73) Titulaire: L'OREAL, 75008 Paris (FR)
(72) Inventeur: CORCUFF, Pierre, F-93330 Neuilly-sur-Marne (FR); LEVEQUE, Jean-Luc, F-93340 Le Raincy (FR)
(74) Mandataire: Peuscet, Jacques
(86) Numéro de dépôt international: FR9200924
(87) Numéro de publication internationale: WO9307522

(56) Documents cités:
- EP-A- 0 382 424
- JOURNAL OF ELECTRON MICROSCOPY TECHNIQUE, vol. 18, 1991, pp. 50-60; J.V. JESTER et al.: 'In vivo, real-time confocal imaging'
- SCANNING, vol. 13, no. 5, septembre 1991, pp. 369-372; K.C. NEW : 'In vivo imaging of human teeth and skin using real-time confocal microscopy'
- PATENT ABSTRACTS OF JAPAN vol. 13, no. 12 (P-812) 12 janvier 1989 ; & JP-A-63 218 911
- EXPERIENTIA, vol. 24, 15 novembre 1968, Basel CH, pp. 1094-1095; D.M. MAURICE : 'Cellular membrane activity in the corneal endothelium of the intact eye'

## Description

L'invention est relative à un appareil pour observer in vivo la structure microscopique de la peau ou d'un tissu similaire, sur une profondeur suffisante depuis la surface extérieure.

On a proposé d'utiliser la microscopie confocale, en temps réel, pour obtenir des images de cellules de tissus in vivo, en particulier pour la cornée de l'oeil. L'article "IN VIVO REAL-TIME CONFOCAL IMAGING" de JAMES V. JESTER, PETER M. ANDREWS, W. MATTHEW PETROLL, MICHAEL A. LEMP, et H. DWIGHT CAVANAGH publié en mai 1991 dans "Journal of Electron Microscopy Technique", 18 (1991) pages 50-60, donne divers exemples sur le sujet. Il n'y est cependant pas question de configuration d'appareil permettant de réaliser des images in vivo de la peau, par microscopie confocale.

L'article "In Vivo Imaging of Human Teeth and Skin Using Real-Time Confocal Microscopy" de NEW et al. publié dans SCANNING, vol. 13, n° 5, septembre 1991, pp. 369-372, montre un appareil permettant d'observer la peau in vivo. Cet appareil comprend un microscope confocal équipé d'un éclairage approprié et d'un disque rotatif muni de trous de diamètre réduit, connu sous le nom de disque de Nipkow, d'un récepteur d'images à haute sensibilité et d'un objectif à immersion. Cet appareil permet d'observer de manière non-invasive la structure de la peau ou d'un tissu similaire. Toutefois, la qualité des images obtenues et la profondeur des observations dans la peau demandent à être améliorées.

On a déjà proposé des appareils à ultrasons ou des appareils utilisant la résonance magnétique nucléaire (RMN) pour observer, in situ, la peau humaine.

La résolution spatiale est relativement limitée, de l'ordre de 100 micromètres, et n'est pas suffisante pour une observation à l'échelle cellulaire.

Autrement dit, les appareils proposés jusqu'à ce jour pour l'observation in vivo de la peau, ne permettent pas de réaliser des observations aussi fines et précises que celles effectuées in vitro, par voie destructive ou invasive.

L'invention a pour but, surtout, de fournir un appareil pour observer in vivo, de manière non-invasive, la structure microscopique de la peau ou d'un tissu similaire, donnant une bonne qualité d'image et une précision comparable à celle obtenue par les méthodes d'observation in vitro, destructives ou invasives. Il est souhaitable, en outre, qu'un tel appareil permette d'effectuer des observations rapides.

Selon l'invention, un appareil pour observer in vivo la structure microscopique de la peau ou d'un tissu similaire comprenant un microscope confocal équipé d'un éclairage approprié, et d'un disque rotatif muni de trous de diamètre réduit, généralement connu sous le nom de disque de Nipkow, d'un récepteur d'images à haute sensibilité et d'un objectif à immersion est caractérisé par le fait qu'il comporte un embout de contact destiné à être placé contre la peau et dans lequel est engagée au moins la partie inférieure dudit objectif, cet embout comportant une ouverture centrale et étant lié à la peau autour de ladite ouverture centrale, l'ensemble étant monté de telle sorte qu'un déplacement relatif axial soit possible entre l'embout de contact et l'objectif.

Pour l'observation, une goutte d'un liquide dont l'indice de réfraction est sensiblement égal à celui de la couche supérieure de la peau (stratum cornéum) est placée dans l'ouverture centrale de l'embout de contact, entre l'objectif et la peau avec lesquels ladite goutte est en contact. Le liquide est avantageusement constitué par une huile d'immersion dont l'indice de réfraction est égal à 1,5.

L'appareil de l'invention, en assurant le maintien de la zone de la peau à observer, et en facilitant la mise en place et la conservation de la goutte de liquide d'immersion, permet d'obtenir des images de qualité.

Avantageusement, l'embout de contact comporte un canal, en particulier capillaire, permettant de réalimenter en liquide ladite ouverture centrale de l'embout de contact.

L'embout de contact peut être lié à la peau à l'aide d'une rondelle adhésive double face comportant un trou central, en correspondance avec l'ouverture centrale de l'embout de contact.

De préférence, l'embout de contact est fixé de manière démontable, en particulier par vissage, sur un bras.

Selon une première possibilité, l'objectif du microscope confocal est porté par un support fixe tandis que l'embout de contact est monté avec possibilité de coulissement relativement à l'objectif.

Selon une autre possibilité, l'objectif peut être déplacé manuellement, en particulier pour atteindre différents sites du corps, ledit objectif étant en outre monté pour pouvoir être déplacé en coulissement relativement à l'embout de contact, immobile sur la peau.

Des moyens de déplacement micrométrique, en particulier actionnés par un moteur électrique pas à pas, sont avantageusement prévus pour commander les déplacements relatifs entre l'objectif et l'embout de contact. Ces moyens permettent en outre des mesures précises des profondeurs atteintes.

L'éclairage du microscope confocal est réalisé en lumière blanche, en particulier à l'aide d'une lampe à mercure filtrée dans la bande 400 nm - 700 nm.

Le récepteur d'images à haute sensibilité est avantageusement constitué par une caméra intensifiée DAGE MTI SIT 68 (10⁻⁵ lux).

L'objectif a un grossissement de l'ordre de 50, et une ouverture numérique (NA) généralement supérieure à 0,7 et de préférence de 0,85.

La distance frontale de l'objectif est au moins égale à 200 micromètres.

L'invention consiste, mises à part les dispositions exposées ci-dessus, en un certain nombre d'autres dispositions dont il sera plus explicitement question ci-après, avec référence aux dessins annexés, à propos d'un exemple de réalisation d'un appareil conforme à l'invention, mais qui n'est nullement limitatif.

La figure 1, de ces dessins, est un schéma de l'appareil conforme à l'invention.

La figure 2 est une vue en plan du disque de Nipkow du microscope confocal de l'appareil de la figure 1.

La figure 3 est une vue en élévation, avec parties coupées, à plus grande échelle, de l'objectif et de l'embout de contact de l'appareil de la figure 1.

La figure 4 est une vue de dessus par rapport à la figure 3, l'objectif étant retiré.

La figure 5 est une vue en plan d'une rondelle adhésive double face.

La figure 6 est une coupe schématique de la peau.

La figure 7, enfin, est un schéma simplifié d'une variante de l'appareil.

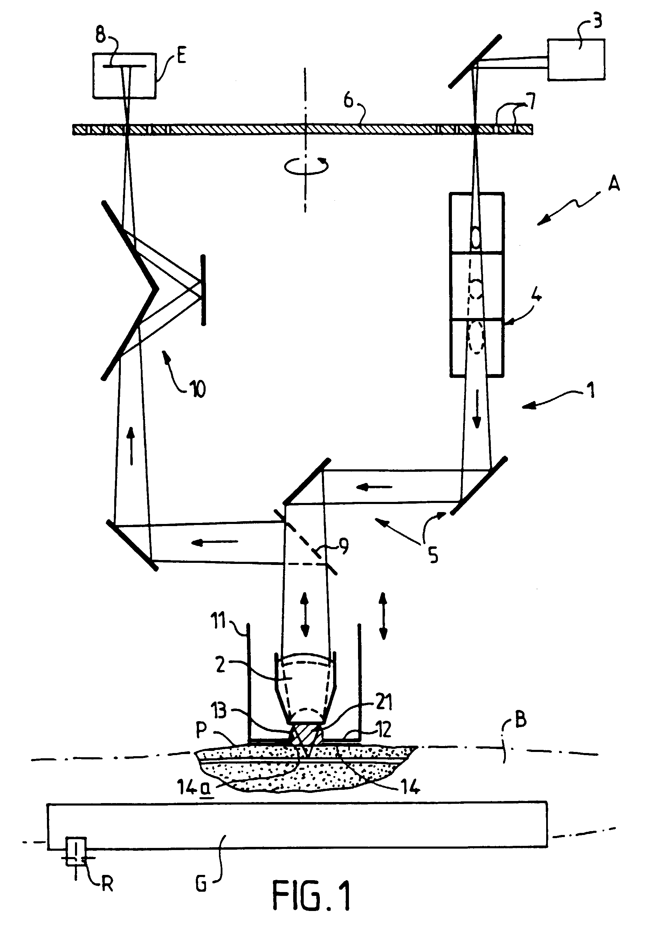

En se reportant aux dessins, notamment à la figure 1, on peut voir un appareil A permettant d'observer in vivo la structure microscopique de la peau. Dans l'exemple considéré, l'appareil A est utilisé pour observer une zone de la peau P du bras B d'un patient.

L'appareil A comprend un microscope confocal 1 par exemple du type TSM TRACOR pour observation en temps réel. Ce microscope 1 est muni d'un objectif 2 à immersion d'huile, dont le grossissement est avantageusement égal à 50 et l'ouverture numérique égale à 0,85. La distance frontale de l'objectif 2 (c'est-à-dire la distance entre sa face de sortie et le foyer) est au moins égale à 200 micromètres. L'éclairage de la zone à observer est assuré par une source 3 de lumière blanche, constituée par exemple par une lampe à mercure, filtrée dans la bande 400-700 nm. Le faisceau de lumière traverse un système inverseur 4 et est renvoyé par un jeu 5 de miroirs, ou moyens équivalents, suivant l'axe optique de l'objectif 2.

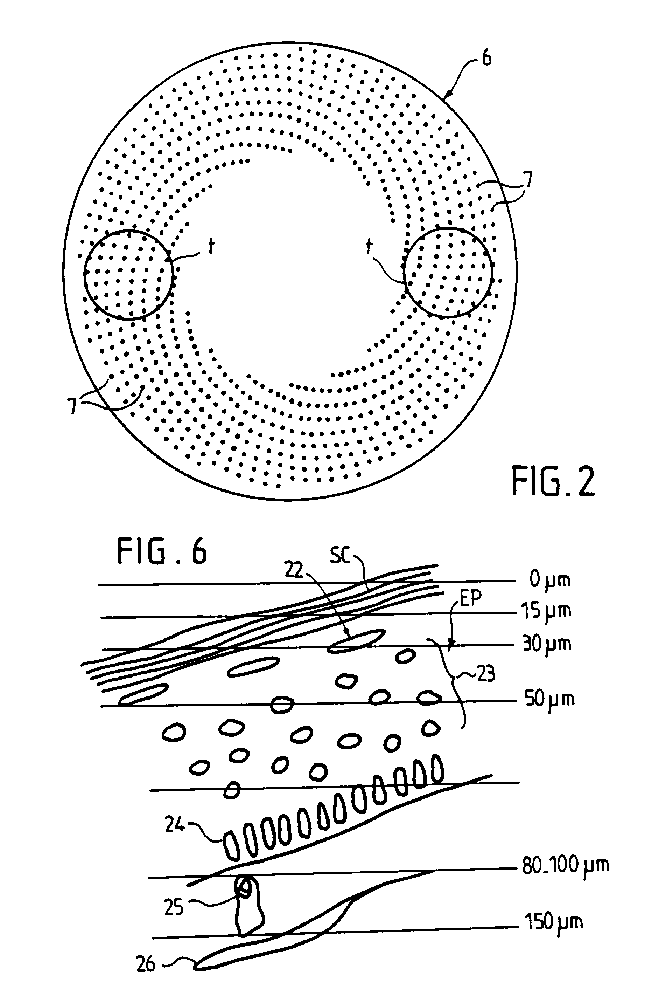

D'une manière connue, pour assurer le balayage de la zone à observer, par un point lumineux, focalisé par l'objectif 2, le faisceau de lumière provenant de la source 3 est haché par un disque 6 comportant une multitude de trous 7 de diamètre réduit, (voir figure 2), judicieusement répartis. Le disque 6 est entraîné en rotation autour d'un axe perpendiculaire à son plan et parallèle à l'axe du faisceau lumineux haché.

Le disque 6 est connu sous le nom de disque de Nipkow.

La lumière renvoyée par la zone à observer est focalisée sur une surface réceptrice 8 à haute sensibilité, en particulier constituée par la surface réceptrice d'une caméra intensifiée E par exemple du type DAGE MTI SIT 68, sensible jusqu'à 10⁻⁵ lux. La lumière renvoyée par la zone à observer est dirigée, vers la surface sensible 8, par un miroir semi-transparent 9, ou équivalent, et par un ensemble de surfaces réfléchissantes 10. Le faisceau renvoyé, avant d'atteindre la surface 8, traverse des trous 7 du disque 6.

Les traces des faisceaux lumineux sur le disque 6 sont schématisés par des cercles t.

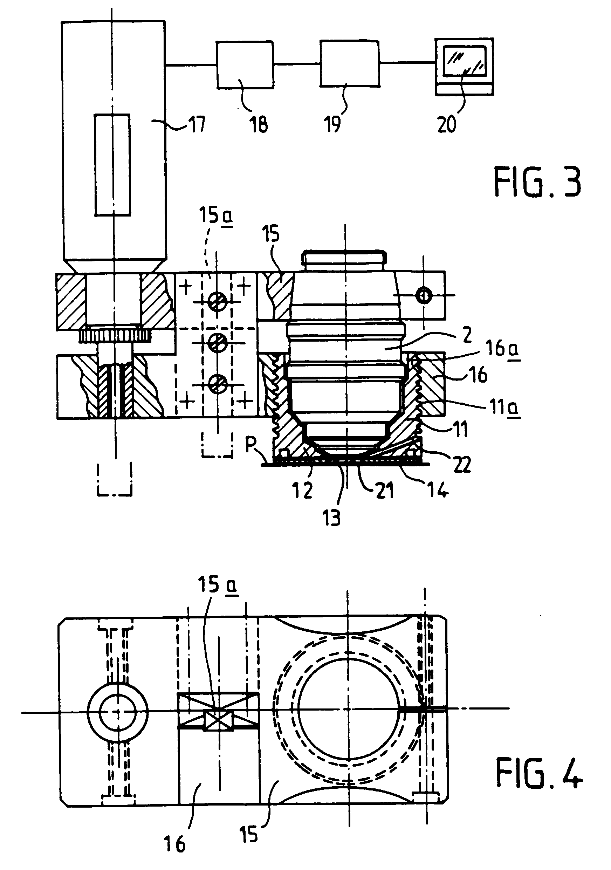

L'appareil A comprend un embout de contact 11 schématiquement représenté sur la figure 1 et montré plus en détail sur la figure 3, constitué par une sorte de manchon comportant un fond 12 muni d'une ouverture centrale circulaire 13 d'un diamètre au moins égal et de préférence supérieur au diamètre de sortie de l'objectif 2. Cet objectif 2, au moins par sa partie inférieure, est engagé dans l'embout 11.

L'embout de contact 11 est destiné à s'appuyer par la face plane inférieure de son fond 12 contre la peau P et à être lié à la peau dans la région annulaire entourant l'ouverture 13.

La liaison entre l'embout 11 et la peau est avantageusement réalisée à l'aide d'une rondelle adhésive double face 14 qui comporte, en son centre, un trou 14a d'un diamètre au moins égal à celui de l'ouverture 13.

L'ensemble est monté de telle sorte qu'un déplacement relatif axial micrométrique soit possible entre l'embout de contact 11 et l'objectif 2.

Dans l'exemple de réalisation de la figure 3, l'objectif 2 est porté par une traverse 15 fixe, elle-même portée par un bâti non représenté. L'embout de contact 11 est porté par un bras 16, parallèle à la traverse 15 et monté coulissant, suivant une direction parallèle à l'axe de l'objectif 2, à l'aide d'un guidage 15a fixé sur la traverse 15. Les déplacements micrométriques du bras 16, et donc de l'embout de contact 11, relativement à la traverse 15 sont assurés par un micromètre de précision 17, porté par la traverse 15, avec vernier au micromètre.

Les déplacements relatifs entre l'embout de contact 11 et l'objectif 2 sont avantageusement pilotés par un moteur pas à pas 18 et un module de commande 19. Cette disposition permet de mesurer avec une précision de 1 micromètre les profondeurs atteintes. Les affichages des profondeurs d'observation, correspondant aux déplacements relatifs de l'embout de contact 11 et de l'objectif 2 peuvent être affichés sur un écran de contrôle 20.

La fixation de l'embout de contact 11 dans le bras 16 est réalisée de manière démontable par vissage d'un filetage extérieur 11a prévu sur l'embout 11, dans un taraudage 16a du bras 16. Il est ainsi possible de changer l'embout 11 et de le choisir selon l'objectif et le site à observer.

En jouant sur l'élasticité de la peau, les déplacements micrométriques de l'embout de contact 11 relativement à l'objectif 2, permettent de faire varier la profondeur des images, constituant des coupes horizontales de la peau, fournies par l'objectif 2. Cette profondeur peut atteindre 150 micromètres et les déplacements possibles de l'embout 11 sont donc d'une amplitude au moins égale à 150 micromètres.

Une goutte 21 d'un liquide dont l'indice de réfraction est sensiblement égal à celui du stratum cornéum (couche superficielle de la peau) est prévue entre la face de sortie de l'objectif 2 et la peau pour supprimer les interfaces et les réflexions de la surface cutanée. Avantageusement, la goutte 21 est une goutte d'huile à immersion d'indice de réfraction égal à 1,5, alors que l'indice de réfraction du stratum cornéum est d'environ 1,5.

Un canal capillaire 22 est prévu dans l'embout 11 et débouche d'une part dans l'ouverture 13 et d'autre part sur la surface périphérique cylindrique de cet embout 11, au-dessous du bras 16. Ce canal capillaire 22 permet de réalimenter l'ouverture 13 en huile d'immersion, par exemple à l'aide d'une seringue, pour maintenir la présence d'une goutte 21 tout au long de la phase d'observation.

Il convient de noter que la rondelle adhésive 14 contribue au maintien du ménisque de la goutte d'huile 21.

L'appareil A comprend un système de gouttière G (voir figure 1) pour maintenir le membre, à savoir le bras B dans l'exemple considéré. La gouttière G peut être articulée sur rotule R pour permettre un ajustement dans un plan horizontal. L'appareil A peut être déplacé verticalement dans son ensemble pour amener l'embout 11 en contact avec la peau.

Il est souhaitable que l'appareil selon l'invention soit mobile au maximum, pour permettre d'atteindre relativement facilement toutes les parties de peau de corps humain.

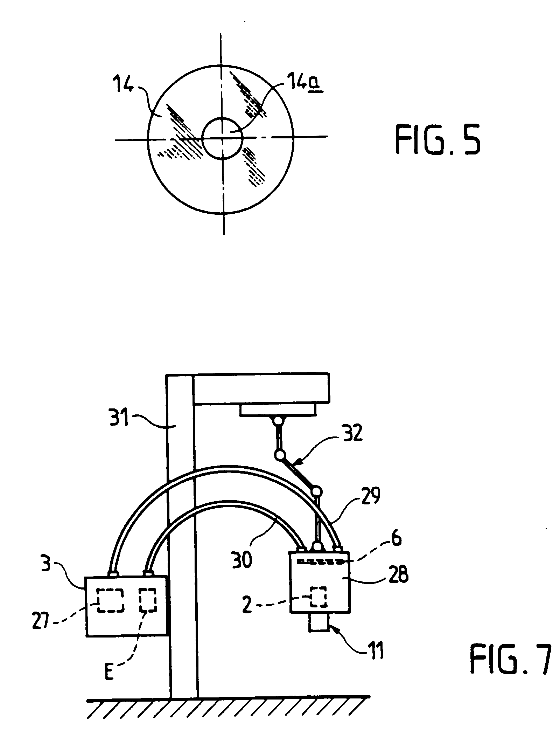

La figure 7 des dessins illustre schématiquement une variante de réalisation visant à donner à l'appareil une grande mobilité.

La source de lumière 3 et la caméra E restent fixes, dans un sous-ensemble ou partie fixe 27 formé par exemple par un boîtier fixé sur un châssis. Les autres éléments de l'appareil A représentés sur la figure 1 forment un sous-ensemble ou partie mobile 28. La liaison optique entre la partie fixe 27 et la partie mobile 28 est assurée par deux faisceaux 29, 30 de fibres optiques souples.

La partie mobile 28 est donc composée de la tête du microscope avec le disque de Nipkow 6, l'objectif 2, l'embout de contact 11 ainsi que les différents éléments optiques 4, 5, 9 et 10 apparaissant sur la figure 1.

L'équipage formé par la partie mobile 28 est suspendu à un mât fixe 31 par l'intermédiaire d'un bras 32 à rotules, permettant l'orientation de la partie mobile 28 dans toutes les directions, et son déplacement pour atteindre toute les parties de la peau du corps humain.

L'éclairage de la tête du microscope est assuré par une lampe à arc de 250 watts, constituant la source 3, par l'intermédiaire des fibres optiques souples 29.

L'image est transmise à distance à la caméra E par l'intermédiaire d'un conduit d'images constitué de la liasse 30 de fibres optiques souples, d'une longueur d'un mètre environ.

Dans une telle variante, lors de l'observation, l'embout de contact 11 reste immobile, tandis que des déplacements micrométriques sont communiqués à l'objectif 2, relativement à cet embout de contact 11.

Dans une autre variante, l'équipage mobile pourrait ne comprendre que l'objectif 2 et le système à contact 11 et serait donc plus léger que dans la variante évoquée précédemment. La liaison équipage mobile/tête de microscope serait assurée par des fibres optiques après correction des trajectoires. Là encore les déplacements micrométriques seraient communiqués à l'objectif 2 alors que l'embout 11 resterait immobile lors de l'observation.

L'image recueillie par la surface sensible 8 peut subir un traitement, après enregistrement sur une bande. Un débruitage peut être réalisé par moyennage en temps réel. L'élimination des bandes dues à la disposition en spirale des trous 7 du disque de Nipkow 6 peut être réalisée par analyse fréquentielle.

Des mesures automatiques des épaisseurs du stratum cornéum et de l'épiderme, par seuillage des noyaux épidermiques peuvent être réalisées. Autrement dit, par observation des images obtenues, on peut déterminer, lors de l'apparition de structures différentes, la transition du stratum cornéum à l'épiderme et de l'épiderme au derme. En repérant l'amplitude du déplacement relatif nécessaire entre objectif 2 et embout de contact 11, pour obtenir ces transitions, on effectue une mesure d'épaisseur relativement précise.

Les mesures d'épaisseur peuvent donner lieu à un moyennage par champs dans quatre cylindres verticaux virtuels de diamètre de 40 micromètres, dont les centres sont situés sur un cercle et décalés angulairement les uns par rapport aux autres de 90°, de manière à corriger une inclinaison éventuelle de la surface observée vis-à-vis de l'axe optique de l'objectif 2.

Une représentation volumique avec recalage des coupes optiques et transparisation à partir de logiciels 3D+ et MIPS peut être effectuée (MISIS-St Etienne).

L'appareil conforme à l'invention permet de réaliser des observations précises et de qualité de la peau in vivo sur une profondeur de 150 micromètres qui atteint, comme illustré sur la figure 6, les capillaires. On peut donc réaliser des coupes optiques, de manière non invasive et non destructive, à travers le stratum cornéum SC, l'épiderme EP (couche granuleuse 22, couche spineuse 23 et couche basale 24) et à travers les capillaires (boucles terminales 25 et grandes anses 26). Les profondeurs moyennes, exprimées en micromètres, à partir de la surface de la peau, ont été indiquées sur la figure 6.

La résolution est inférieure au micromètre et couvre un champ d'observation de 300 micromètres de diamètre. L'appareil travaille en temps réel ce qui permet de travailler dans un espace 4D (trois dimensions + temps).

L'embout de contact 11 lié à la peau permet d'immobiliser le champ d'observation en s'affranchissant des mouvements incontrôlés du patient, suivant deux directions X, Y, ce qui contribue à l'obtention de bonnes images. L'embout 11, fixé de manière démontable sur le bras 16 est interchangeable et sa forme peut être adaptée au site anatomique.

La liaison entre la face inférieure du fond 12 de l'embout et la peau permet de limiter la pression du contact sur la peau et d'éliminer le battement artériel. Cette liaison permet de maintenir le ménisque d'huile et de réaliser des mesures exactes d'épaisseur.

Suivant la réalisation choisie, concernant les mesures des différentes couches épidermiques, dont la reproductibilité peut être affectée par les mouvements du patient et en particulier les mouvements dans le sens vertical, il a été conçu d'effectuer l'enregistrement video de la zone mesurée avec une vitesse de déplacement vertical d'au moins µm/seconde. Pour chaque enregistrement, l'altitude de la couche enregistrée est indiquée. Grâce à une carte de mixage video, il est ensuite possible de superposer les enregistrements d'images qui ont été pris au même niveau de profondeur dans la peau, permettant ainsi d'obtenir par ce procédé de superposition, des images plus nettes et d'obtenir également une altitude de pénétration plus élevée.

## Revendications

1. Appareil pour observer in vivo la structure microscopique de la peau ou d'un tissu similaire, comprenant un microscope confocal (1) équipé d'un éclairage approprié, et d'un disque rotatif (6) muni de trous de diamètre réduit, généralement connu sous le nom de disque de Nipkow, d'un récepteur d'images (8) à haute sensibilité et d'un objectif (2) à immersion, caractérisé par le fait qu'il comporte un embout de contact (11) destiné à être placé contre la peau et dans lequel est engagée au moins la partie inférieure dudit objectif (2), cet embout (11) comportant une ouverture centrale (13) et étant lié à la peau autour de ladite ouverture centrale, l'ensemble étant monté de telle sorte qu'un déplacement relatif axial soit possible entre l'embout de contact (11) et l'objectif (2).

2. Appareil selon la revendication 1, caractérisé par le fait que l'embout de contact (11) est lié à la peau à l'aide d'une rondelle (14) adhésive double face comportant un trou central (14a), en correspondance avec l'ouverture centrale (13) de l'embout de contact (11).

3. Appareil selon la revendication 1 ou 2, caractérisé par le fait que l'embout de contact (11) est fixé de manière démontable, en particulier par vissage, sur un bras (16).

4. Appareil selon l'une des revendications précédentes, caractérisé par le fait que l'objectif (2) du microscope confocal est porté par un support fixe (15) tandis que l'embout de contact (11) est monté avec possibilité de coulissement relativement à l'objectif.

5. Appareil selon la revendication 1 ou 2, caractérisé par le fait qu'il comprend une partie fixe (27) comportant une source de lumière (3) et une caméra (E) et une partie mobile (28) composée de la tête du microscope avec le disque de Nipkow (6), l'objectif (2) et l'embout de contact (11), la liaison optique entre la partie fixe (27) et la partie mobile (28) étant assurée par deux faisceaux (29, 30) de fibres optiques souples.

6. Appareil selon la revendication 5, caractérisé par le fait que la partie mobile (28) est suspendue à un mât fixe (31) par l'intermédiaire d'un bras (32) à rotules.

7. Appareil selon la revendication 4 ou 5, caractérisé par le fait que des moyens (17) de déplacement micrométrique, en particulier actionnés par un moteur électrique pas à pas (18), sont prévus pour commander les déplacements relatifs entre l'objectif (2) et l'embout de contact (11).

8. Appareil selon la revendication 1, caractérisé par le fait qu'au cours de l'observation, une goutte (21) d'un liquide dont l'indice de réfraction est sensiblement égal à celui de la couche supérieure de la peau (stratum corneum) est placée dans l'ouverture centrale (13) de l'embout de contact (11).

9. Appareil selon la revendication 8, caractérisé par le fait que le liquide est constitué par une huile d'immersion dont l'indice de réfraction est égal à 1,5.

10. Appareil selon la revendication 8 ou 9, caractérisé par le fait que l'embout de contact (11) comporte un canal (22), en particulier capillaire, permettant de réalimenter en liquide ladite ouverture centrale (13) de l'embout de contact (11).

## Patentansprüche

1. Vorrichtung zur In-Vivo-Beobachtung der mikroskopischen Struktur der Haut oder eines ähnlichen Gewebes, bestehend aus einem konfokalen Mikroskop (1), das mit einer geeigneten Beleuchtung, mit einer allgemein unter dem Namen Nipkowscheibe bekannten rotierenden Scheibe (6), die mit Löchern mit kleinem Durchmesser versehen ist, mit einem Bildempfänger (8) mit hoher Empfindlichkeit und mit einem Immersionsobjektiv (2) ausgerüstet ist, dadurch gekennzeichnet, daß sie einen Kontaktaufsatz (11) aufweist, der dazu bestimmt ist, auf die Haut aufgesetzt zu werden, und in den zumindest der untere Teil des Mikroskops (2) eingesteckt ist, wobei der Aufsatz (11) eine zentrale Öffnung (13) aufweist und um die zentrale Öffnung herum mit der Haut verbunden ist sowie die Einheit derart montiert ist, daß eine axiale Relativbewegung zwischen dem Kontaktaufsatz (11) und dem Objektiv (2) möglich ist.

2. Vorrichtung nach Anspruch 1, dadurch gekennzeichnet, daß der Kontaktaufsatz (11) mit der Haut durch eine zweiseitige Klebescheibe (14) verbunden ist, die ein mit der zentralen Öffnung (13) des Kontaktaufsatzes (11) übereinstimmendes zentrales Loch (14a) aufweist.

3. Vorrichtung nach Anspruch 1 oder 2, dadurch gekennzeichnet, daß der Kontaktaufsatz (11) an einem Arm (16), insbesondere durch Verschrauben, lösbar befestigt ist.

4. Vorrichtung nach einem der vorhergehenden Ansprüche, dadurch gekennzeichnet, daß das Objektiv (2) des konfokalen Mikroskops von einem feststehenden Halter (15) getragen ist, während der Kontaktaufsatz (11) relativ zum Objektiv verschiebbar montiert ist.

5. Vorrichtung nach Anspruch 1 oder 2, dadurch gekennzeichnet, daß sie einen feststehenden Teil (27), der eine Lichtquelle (3) und eine Kamera (E) aufweist, und einen beweglichen Teil (28) umfaßt, der den Kopf des Mikroskops mit der Nipkowscheibe (6), dem Objektiv (2) und dem Kontaktaufsatz (11) aufweist, wobei die optische Verbindung zwischen dem feststehenden Teil (27) und dem beweglichen Teil (28) durch zwei Bündel (29, 30) von biegsamen optischen Fasern gewährleistet ist.

6. Vorrichtung nach Anspruch 5, dadurch gekennzeichnet, daß der bewegliche Teil (28) über einen Arm (32) mit Kugelgelenken an einer feststehenden Stange (31) aufgehängt ist.

7. Vorrichtung nach Anspruch 4 oder 5, dadurch gekennzeichnet, daß eine insbesondere durch einen elektrischen Schrittmotor (18) betätigte Mikrometerbewegungseinrichtung (17) zur Steuerung der Relativbewegungen zwischen dem Objektiv (2) und dem Kontaktaufsatz (11) vorgesehen ist.

8. Vorrichtung nach Anspruch 1, dadurch gekennzeichnet, daß während der Beobachtung ein Tropfen (21) einer Flüssigkeit, deren Brechzahl im wesentlichen gleich der der obersten Schicht der Haut (Stratum corneum) ist, in der zentralen Öffnung (13) des Kontaktaufsatzes (11) angeordnet wird.

9. Vorrichtung nach Anspruch 8, dadurch gekennzeichnet, daß die Flüssigkeit aus Immersionsöl besteht, dessen Brechzahl gleich 1,5 ist.

10. Vorrichtung nach Anspruch 8 oder 9, dadurch gekennzeichnet, daß der Kontaktaufsatz (11) einen Kanal (22), insbesondere einen Kapillarkanal, aufweist, der die Nachversorgung der zentralen Öffnung (13) des Kontaktaufsatzes (11) mit Flüssigkeit gestattet.

## Claims

1. Apparatus for in vivo observation of the microscopic structure of the skin or of a similar tissue, comprising a confocal microscope (1) equipped with an appropriate illumination, and with a rotary disk (6) provided with reduced diameter holes, generally known under the name of Nipkow disk, with a highly sensitive image receiver (8) and with an immersion lens (2), characterized in that it includes a contact endpiece (11) intended to be placed against the skin and in which is engaged at least the lower part of said lens (2), this endpiece (11) including a central opening (13) and being linked to the skin about said central opening, the whole being mounted in such a manner that a relative axial displacement is possible between the contact endpiece (11) and the lens (2).

2. Apparatus according to Claim 1, characterized in that the contact endpiece (11) is linked to the skin by means of a double-face adhesive washer (14) including a central hole (14a), in correspondence with the central opening (13) of the contact endpiece (11).

3. Apparatus according to Claim 1 or 2, characterized in that the contact endpiece (11) is fixed in a detachable manner, especially by screwing, onto an arm (16).

4. Apparatus according to one of the preceding claims, characterized in that the lens (2) of the confocal microscope is carried by a fixed support (15), while the contact endpiece (11) is mounted with the possibility of sliding relative to the lens.

5. Apparatus according to Claim 1 or 2, characterized in that it comprises a fixed part (27) including a light source (3) and a camera (E) and a movable part (28) composed of the head of the microscope together with the Nipkow disk (6), the lens (2) and the contact endpiece (11), the optical linkage between the fixed part (27) and the movable part (28) being assured by two bundles (29, 30) of flexible optical fibres.

6. Apparatus according to Claim 5, characterized in that the movable part (28) is suspended from a fixed mast (31) via an arm (32) with pivot couplings.

7. Apparatus according to Claim 4 or 5, characterized in that micrometric displacement means (17), especially activated by a stepping electric motor (18), are provided to control the relative displacements between the lens (2) and the contact endpiece (11).

8. Apparatus according to Claim 1, characterized in that in the course of the observation a drop (21) of a liquid, the refractive index of which is substantially equal to that of the upper layer of the skin (stratum corneum), is placed in the central opening (13) of the contact endpiece (11).

9. Apparatus according to Claim 8, characterized in that the liquid is composed of an immersion oil, the refractive index of which is equal to 1.5.

10. Apparatus according to Claim 8 or 9, characterized in that the contact endpiece (11) includes a channel (22), especially a capillary channel, permitting the resupply of liquid to said central opening (13) of the contact endpiece (11).
